Europäisches Patentamt

**European Patent Office**

**Office européen des brevets**

(19)

(11) Publication number: **0 154 530**
**A2**

(12) # EUROPEAN PATENT APPLICATION

(21) Application number: **85301422.3**

(22) Date of filing: **01.03.85**

(51) Int. Cl.⁴: **A 61 K 35/78**

(30) Priority: **01.03.84 ZA 841550**

(43) Date of publication of application:
**11.09.85 Bulletin 85/37**

(84) Designated Contracting States:
**AT BE CH DE FR GB IT LI LU NL SE**

(71) Applicant: **Robinson, Reuben Judah**
**St. Albans Institute Linton Grange**
**Port Elizabeth(ZA)**

(72) Inventor: **Robinson, Reuben Judah**
**St. Albans Institute Linton Grange**
**Port Elizabeth(ZA)**

(74) Representative: **Grundy, Derek George Ritchie et al,**
**CARPMAELS & RANSFORD 43, Bloomsbury Square**
**London WC1A 2RA(GB)**

(54) **Medicinal substance.**

(57) A medicinal substance for the treatment of various illnesses including cancer, cell damage caused by harmful rays, such as X- and gamma rays, etc., which is derived from the plant Psidium guajava Linn.

EP 0 154 530 A2

Croydon Printing Company Ltd.

## MEDICINAL SUBSTANCE

THIS invention relates to a medicinal substance and its method of preparation.

According to the invention a medicinal substance is provided which includes an extract of the fruit of the plant Psidium guajava Linn.

Further according to the invention a medicinal substance is provided for the treatment of cancer which is derived from the plant Psidium guajava Linn.

Details of the constituents of the fruit of Psidium guajava Linn are, for example, given on page 957 of "Hagers Handbuch der Pharmazeutischen Praxis" - Vollständige (Vierte) Neuausgabe - Springer - Verlag - 1977. A free translation of the relevant passage reads as follows:

"Fructus Psidii.  Guajven. Guaven.

Pear or apple shaped fruit which tastes somewhat like pears and figs.

Ingredients: 0.02 to 1.2% of ascorbic acid; in the unripe fruit much quercetine.

Guajaverin, gallic acid, 0.1%leucocyanidien, 0.1% of arabinose hexahydroxydiphenic acid esters and little free ellagic acid, oxalate; in ripe fruit much free ellagic acid and little leucocyanidine.

According to MISRA and SESHADRI (Phytochemistry 7,641 (1968)) the fruit contains carotine, vitamins $B_1$, $B_2$, $B_6$, glucose, fructose, sacharose and water soluble arabane. According to SENGUPTA et al (Australian J. Chemistry 18,851 (1965)) arabinane which during hydrolysis produces galacturonic acid, 14% galactose, 59,4% arabinose with traces of xylose and rhamnose. According to FERRO et al (Tecnologia 11,30 (1969) about 6% pectine. In the seeds 10% of fatty oil with 15% saturated fatty acids and oilic and linolic acid as the main fatty acids. In the volatile components of the fruit and according to TORLIKE et al (Lebensmittel Wiss. - Technologie 6, 32 (1973) alcohols (M. A., $\alpha$ - Terpineol and others) ketones ( $\beta$ -ionone, acetone, methylisopropyl ketone), aldehyde (butanal, benzaldehyde, citral and others), ester (methylcinnamate, cinnamylacetate and others, hydrocarbons (benzene, limones, caryophyles and others) and according to CHAN et al (J. Food Sci. 36, 237 (1971)), citric-, malonic-, lactic - and galakturonic-acids are present. Furthermore a saponine which after hydrolysis provides oleanolic acid and $\beta$-sitosterine, is present (VARSHNEY et al: Chem. Abstr. 77, 85583 (1972))."

It will of course be appreciated that the precise chemical breakdown of the fruit may vary from one variety of fruit to the next and often also from one fruit to the next of the same variety.

- 4 -

Applicant does not know which of the aforesaid substances in fact constitute the active ingredients of the medicinal substance of the invention, but he believes that it may well comprise a combination of several of them.

The extract according to the invention may be obtained in any suitable manner but preferably it includes the step of treating the fruit with a suitable solvent such as a suitable ketone and/or alcohol, and/or propylene glycol, and/or glycerine, and/or benzene, and/or methylcinnamate.

In a preferred form of the invention the solvent comprises acetone.

Applicant has found the medicinal substance according to the invention effective in the treatment, and the prevention, of a variety of different diseases and illnesses, some of which belong to the group of so called "incurable" diseases such as cancer, for example.

Applicant has also found that because of its ability

- 5 -

to assist the body in getting rid of infected body cells, the substance is also suited for use as a tonic for improving the general health of humans.

The substance has furthermore also been found suitable for the treatment of cell damage caused to the body of humans and/or animals by harmful rays such as X-rays, gamma-rays etc.

The medicinal substance according to the invention may be administered orally, intravenously or topically depending on the particular type of disease or illness being treated. It will be appreciated that the particular mode of administration will determine the form and concentration in which the medicinal substance has to be utilised.

In one embodiment of a method for preparing a medicinal substance according to the invention, which will now be described by way of example only, a quantity of fruit of Psidium guajava Linn (of the type commonly known as the Medeira guava) is peeled, the pips removed, and the residue heated to boiling

- 6 -

with approximately 2kg of sugar to every 100kg of
fruit. The product is allowed to simmer for
approximately one hour and then allowed to cool.

A quantity of acetone equal in volume to that of the
aforesaid product is added thereto and the mixture
allowed to stand over for a period of time,
preferably for at least twelve hours.

The fibres and other solid material are removed from
the products in any suitable manner, such as by
means of filtration, and the resulting liquid
component is then heated under vacuum until its
volume has been reduced to approximately one sixth
of its original volume. The resulting liquid, which is
substantially completely acetone-free, has a clear amber
colour.

Instead of the aforesaid final heating step, the
liquid component may be ignited to cause the burning
off of approximately 90% of the acetone. In this
case the resulting liquid, which is suited for external
application only, has a dark brown colour. If required, the
liquid may be heated further to remove the remaining acetone.
Applicant has found that as far as the treatment of
malignant cancers are concerned, the best results

- 7 -

                                    acetone-free
are obtained if the aforesaid/resulting liquid, which comprises the medicinal substance according to the invention, is administered orally in an average dose of 30 ml three times per day.


In the case of lung cancer, applicant has found that on an average a total amount of approximately 1500 ml of such liquid, administered in the aforesaid manner, is required to cure a patient.


In the case of human cell damage caused by excessive exposure to X-rays and/or gamma-rays, applicant has found that on an average between 1000 ml and 3000 ml
                                  acetone-free
of the aforesaid resulting/liquid given at a dosage of 30 ml three times per day is required to cure a patient.


Applicant has found the aforesaid resulting liquid to remain stable and fungus-free over very long periods of time, even if kept at very high or very low temperatures.


For an oral application, the aforesaid resulting acetone-free liquid comprising the medicinal substance according to the invention may be utilised as such, or it may be mixed with a suitable flavouring agent such as lemon syrup, for example, which results in a drink with a wine-like taste. Applicant has also found that a drink with a particularly pleasing taste results if the medicinal substance according to the invention is mixed with wine.

In the case of the treatment of non-malignant skin cancer, the aforesaid resulting acetone-free liquid (preferably mixed with 10% acetone by volume) or the aforesaid resulting liquid which resulted from the burning off of 90 percent of the acetone, may be applied as such to the area being treated.
Such treatment may, for example, be carried out by locating a dressing of an absorbent material over the area being treated and then covering it with a second dressing of a non-absorbent material. Further applications of medicinal substance may then be effected by putting a few dropsof the substance into the absorbent dressing, say, by means of a syringe or the like, to keep the absorbent material saturated.

Although applicant has found that skin cancers may

- 9 -

be cured  by means of such treatment in a few days, he believes it advisable that the treatment be continued for at least equal the period of time which was required for the treated area to appear visably cured.

In the case of other forms of cancer it is also believed advisable for the treatment to be continued for equal the period of time required for curing the illness.

Apart from numerous actual successful treatments of humans, and animals such as dogs, suffering from various types of cancers with the medicinal substance according to the invention, the applicant has also performed the following test in the laboratory:

Forty mice were injected with a carcinogenic material and within six weeks twenty eight had developed large tumors.  All the mice lost their hair on their shoulders, face, and ears, and some on other parts of their body.  They were listless, their eyes were dull, and visually they were very

- 10 -

sick. Fourteen of the mice with tumors and six of the others were separated and exposed to a sole supply of drinking water which contained, on a volume per volume basis, 25% of the aforesaid acetone-free resulting liquid comprising the substance according to the invention.

After ten weeks these mice had grown hair and by appearance they were cured. By this time all the untreated mice had died.

In an attempt to test the medicinal substance according to the invention for any possible harmful effects on humans and/or animals, applicant himself over a period of 100 days consumed approximately 15 liters of the aforesaid/resulting liquid without
(acetone-free)
noticing any side effects. In the case of mice, which were given orally 390 mls of the same substance over a period of 120 days, a dosage which, considering the weight ratio between humans and mice, is approximately thirty times the volume which the applicant found is required on an average to cure lung cancer in a human, the only side effect was a temporary occurence of premature births which

only lasted for a couple of months.  The premature born mice showed no signs of deformity.

It will be appreciated that the invention provides a medicinal substance which can provide a major breakthrough in the treatment of a variety of diseases especially hitherto believed incurable diseases and illnesses.

It will be appreciated further that there are many variations in detail possible with a medicinal substance, and its method of preparation, according to the invention without departing form the scope of the appended claims.

- 12 -

CLAIMS

1.

A medicinal substance including an extract of the fruit of the plant Psidium guajava Linn.

2.

A medicinal substance for the treatment of cancer which is derived from the plant Psidium guajava Linn.

3.

A medicinal substance for the treatment of cancer including an extract of the fruit of the plant Psidium guajava Linn.

4.

The medicinal substance of claims 1 or 3 wherein the extract is obtained by treating the fruit with a suitable solvent.

4a.

The medicinal substance of Claim 4 wherein the solvent is a suitable ketone and/or alcohol, and/or propylene glycol, and/or glycerine, and/or benzene, and/or methylcinnamate.

- 13 -

5.

The medicinal substance of claim 4a wherein the ketone comprises acetone.

6.

A method of preparing a medicinal substance by heating a quantity of the fruit of Psidium guajava Linn, which have been peeled and the pips removed, to boiling with approximately 2kg of sugar to every 100kg of fruit; adding thereto a quantity of acetone equal in volume to that of the product; allowing the mixture to stand over for a period of time; removing the fibres and any other solid materials; and heating the resulting liquid component under vacuum to reduce its volume.

7.

The method of claim 6 wherein the heating under vacuum is continued until the volume of the resulting liquid component referred to has been reduced to approximately one sixth of its original volume and is substantially acetone-free.

8.

The method of claim 6 wherein, instead of the final heating step, the liquid component of the product is ignited to cause the burning off of approximately 90% of the acetone.

9.

The medicinal substance of any one of claims 4, 4a or 5 in so far as they are directly or indirectly dependant on claim 1, or the product of the methods of any one of claims 6 to 8, for the treatment of cancer.

10.

The medicinal substance of any one of claims 1, 4, 4a (in so far as they are dependant on claim 1), 5, or the product of the methods of any one of claims 6 to 8, for use as a tonic for human beings.

11.

The medicinal substance of any one of claim 1, 4, 4a (in so far as they are dependant on claim 1), 5, or the product of the methods of anyone of claims 6 to 8, for the treatment of cell damage caused to humans and/or animals by harmful rays such as X-rays, gamma rays etc.

12.

A substance derived from the plant <u>Psidium</u> <u>guajava</u> Linn. for use in medicine.

13.

An extract of the fruit of the plant <u>Psidium</u> <u>guajava</u> Linn. for use in medicine.

14.

A pharmaceutical composition comprising a medicinal substance according to any one of Claims 1 to 5 or 9 to 11 in combination with a physiologically acceptable excipient, diluent, or carrier.

15.

A method of producing a pharmaceutical composition comprising formulating a medicinal substance according to any one of Claims 1 to 5 or 9 to 11 with a physiologically acceptable excipient, diluent, or carrier.